Europäisches Patentamt

European Patent Office

Office européen des brevets

(11) Numéro de publication: **0 377 342**

**A1**

## DEMANDE DE BREVET EUROPEEN

(21) Numéro de dépôt: **89400001.7**

(22) Date de dépôt: **02.01.89**

(51) Int. Cl.5: **A62B 23/06, G02C 11/00, A61F 9/02**

(43) Date de publication de la demande:
**11.07.90 Bulletin 90/28**

(84) Etats contractants désignés:
**AT BE CH DE ES FR GB GR IT LI LU NL SE**

(71) Demandeur: **Walti, Jean-Jacques**
**20 rue Ernest Renan**
**F-92130 Issy-les-Moulineaux(FR)**

(72) Inventeur: **Walti, Jean-Jacques**
**20 rue Ernest Renan**
**F-92130 Issy-les-Moulineaux(FR)**

(74) Mandataire: **Portal, Gérard et al**
**Cabinet Beau de Loménie 55, rue**
**d'Amsterdam**
**F-75008 Paris(FR)**

(54) **Dispositif de lutte contre la pollution.**

(57) La présente invention concerne un dispositif de lutte contre la pollution, caractérisé en ce qu'il est constitué d'un support sur lequel sont fixées deux branches (4, 4') dont chacune porte, à son extrémité, un filtre (5, 5') convenable que l'on peut positionner à l'entrée de chaque narine.

EP 0 377 342 A1

## Dispositif de lutte contre la pollution.

La présente invention concerne un dispositif de lutte contre la pollution et plus particulièrement un dispositif de protection contre ladite pollution.

On sait que l'homme, plus particulièrement le citadin, est soumis quotidiennement et de façon de plus en plus intense à des pollutions de toutes sortes. Ces pollutions sont constituées soit par des substances chimiques les plus diverses (gaz, particules...) indûment libérées par les chaudières, les moteurs..., soit même par des microbes qui se développent dans les bouillons de culture qui constituent les transports urbains, etc

Il est donc souhaitable de pouvoir disposer d'un dispositif individuel permettant à chacun de lutter contre ces pollutions.

On a préconisé notamment dans ce domaine l'utilisation de masques ; l'expérience a montré que le succès rencontré par ces masques n'était pas très étendu.

On préconise donc, et cela constitue l'objet de la présente invention, un dispositif rendu solidaire d'un support fixable sur la tête, caractérisé en ce qu'il comporte des filtres convenables rendus solidaires desdits supports et que l'on peut positionner à l'entrée de chaque narine.

Parmi les supports utilisables, on peut citer par exemple des lunettes ; comme les lunettes constituent en fait le support le plus commode, nous décrirons dans ce qui suit l'invention en se référant auxdites lunettes.

Sur lesdites lunettes on fixe, de part et d'autre du nez, deux branches terminées chacune par un filtre ou un dispositif porte-filtre. La fixation desdites branches sur les lunettes peut être fixe, mais elle est de préférence mobile grâce à l'utilisation d'une rotule qui permet de mettre en place le filtre sous la narine ou de bagues dans lesquelles chaque branche terminée par un filtre peut coulisser.

Les filtres utilisables dans la présente invention sont bien connus des spécialistes ; il s'agit de n'importe quel filtre dont on sait que, parcouru par un courant d'air, il retient les gaz, les particules ou mêmes les microbes dont on veut débarrasser ledit courant d'air. Parmi les filtres utilisables, on citera par exemple les filtres du type de ceux utilisés dans les embouts filtrants de cigarettes. Mais, il est aussi possible d'imprégner lesdits filtres avec des produits adéquats, par exemple des produits bactéricides.

Les filtres peuvent être fixés à demeure à l'extrémité de chacune des branches solidaires des lunettes, mais ils peuvent également et de préférence être montés dans des dispositifs connus permettant un échange des filtres lorsque le besoin s'en fait sentir.

On a indiqué précédemment que les lunettes utilisées pouvaient être quelconques. Il est souvent intéressant de munir lesdites lunettes de verres de protection et même, dans les cas extrêmes, de rendre ces lunettes étanches autour des yeux de ceux qui les portent.

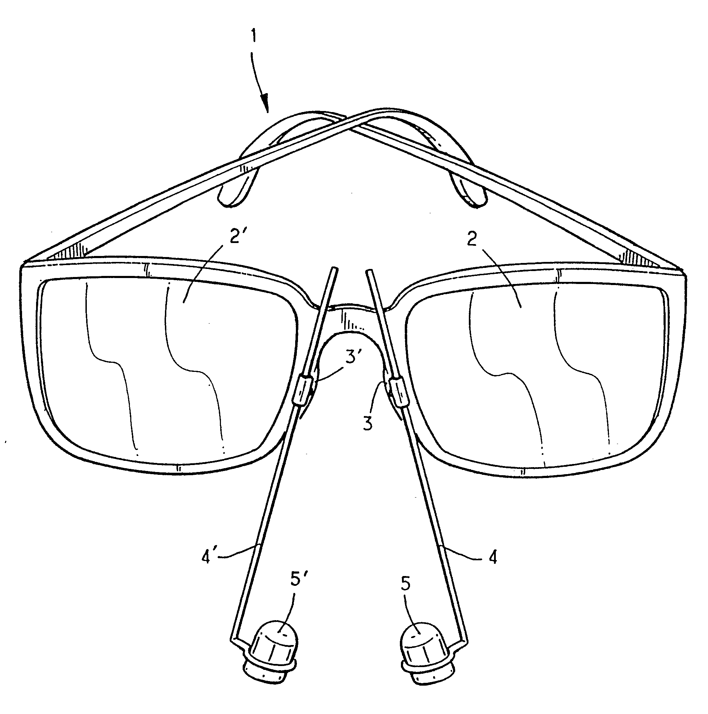

L'exemple non limitatif suivant illustre l'invention ; cet exemple fait référence à la figure unique.

Sur cette figure on a représenté :

- en 1, une paire de lunettes comportant notamment des verres 2,2´ et un pince-nez 3,3´,

- en 4 et 4´, deux branches fixées chacune sur ledit pince-nez 3,3´ ; on a vu que la fixation des branches 4 et 4´ sur le pince-nez 3,3´ pouvait avantageusement être constituée par un élément à rotule permettant de faire pivoter chaque branche par rapport aux lunettes et/ou d'une bague permettant de faire glisser chaque branche selon son axe.

- en 5 et 5´, des filtres qui sont disposés à l'extrémité des branches 4 et 4´ et qui peuvent être positionnés, grâce à la relative mobilité desdites branches 4 et 4´, à l'entrée de chaque narine.

## Revendications

1. Dispositif de lutte contre la pollution, caractérisé en ce qu'il est constitué d'un support sur lequel sont fixées deux branches dont chacune porte, à son extrémité, un filtre convenable que l'on peut positionner à l'entrée de chaque narine.

2. Dispositif selon la revendication 1, caractérisé en ce que ledit support est constitué par des lunettes.

3. Dispositif selon la revendication 2, caractérisé en ce que chaque branche est rendue solidaire desdites lunettes par l'intermédiaire d'un dispositif à rotule et/ou coulissement.

4. Dispositif selon l'une des revendications précédentes, caractérisé en ce que ledit filtre est porté par un porte-filtre de façon à être changé.

5. Dispositif selon l'une des revendications 2, 3 et 4, caractérisé en ce que lesdites lunettes sont protectrices pour les yeux.

6. Dispositif selon la revendication 5, caractérisé en ce que lesdites lunettes comportent des verres protecteurs.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | FR-A-2169629 (WORLD INVENTIONS)<br>* page 1, ligne 1 - ligne 4 *<br>* page 1, ligne 27 - ligne 33 *<br>* page 5, ligne 1 - ligne 13; figures 7, 8 *<br>--- | 1, 2, 4, 5, 6 | A62B23/06<br>G02C11/00<br>A61F9/02 |
| X | GB-A-24397/1912 (LOUCH)<br>* page 2, ligne 6 - ligne 13 *<br>* page 2, ligne 30 - ligne 39; figure 1 * | 1, 4 | |
| A | | 2 | |
| | --- | | |
| A | US-A-1443820 (HUDSON)<br>* page 1, ligne 10 - ligne 18; figures 1, 2 *<br>--- | 1, 2, 4, 5, 6 | |
| A | WO-A-8703704 (TIMMONS)<br>* abrégé; figure 1 *<br>----- | 1, 2, 3 | |

DOMAINES TECHNIQUES
RECHERCHES (Int. Cl.5 )

A62B
G02C
A61F

Le présent rapport a été établi pour toutes les revendications

| Lieu de la recherche | Date d'achèvement de la recherche | Examinateur |
|---|---|---|
| LA HAYE | 08 SEPTEMBRE 1989 | WALVOORT B.W. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
........................................................................
& : membre de la même famille, document correspondant

EPO FORM 1503 03.82 (P0402)